Europäisches Patentamt

European Patent Office

Office européen des brevets

(11) Publication number: **0 121 901**
**B1**

(12) **EUROPEAN PATENT SPECIFICATION**

(45) Date of publication of patent specification: **16.03.88**

(51) Int. Cl.⁴: **A 61 K 31/505,** A 61 K 9/22

(21) Application number: **84103698.1**

(22) Date of filing: **04.04.84**

(54) pH independent controlled releasable tablets.

(30) Priority: **08.04.83 US 483285**

(43) Date of publication of application:
**17.10.84 Bulletin 84/42**

(45) Publication of the grant of the patent:
**16.03.88 Bulletin 88/11**

(84) Designated Contracting States:
**AT BE CH DE FR GB IT LI LU NL SE**

(56) References cited:
EP-A-0 074 584
CH-A- 595 841
US-A-4 361 546
US-A-4 367 217

MARTINDALE, The Extra Pharmacopoeia, 28th
Edition, London 1982, Pages 950, 951, 958, 959

(73) Proprietor: **Boehringer Ingelheim Ltd.**
**90 East Ridge P.O. Box 368**
**Ridgefield, Conn 06877 (US)**

(72) Inventor: **Park, Jung-Yong, Dr.**
**3 Deal Drive**
**Danbury Conn. 06810 (US)**
Inventor: **Debski, Jerzy**
**Hillcrest Apartments Clapboard Ridge Road**
**Danbury Conn. 06810 (US)**
Inventor: **Mooney, Kieran G., Dr.**
**Hattertown Road**
**Newtown Conn. 06470 (US)**

(74) Representative: **Milnes, Rodger et al**
**Boehringer Ingelheim Zentrale GmbH ZA**
**Patente Postfach 200**
**D-6507 Ingelheim am Rhein (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 121 901

**Description**

U.S. Patent No. 3,031,450 describes 2,6-bis-(diethanolamino)-4,8-dipiperidino-pyrimido [5,4-d] pyrimidine or dipyridamole as a coronary vasodilator and it has been used in various pharmaceutical forms for many years. However, dipyrimidole is readily water-soluble only in an acidic medium, and therefore it can only go into solution from solid pharmaceutical forms and then be absorbed if the pharmaceutical preparations remain in the acidic range for a sufficiently long period. Thus, the solubility and hence also the absorption greatly depend upon the retention time and the pH value in the stomach and upper intestinal tract. This results in sharp inter-individual and intra-individual fluctuations in the blood levels (see Table 3 below) since the mobility of a patient, the pH of the patient's stomach and intestines, and the patient's food intake have a considerable influence on absorption. In some patients, the blood levels may even be so low that they practically amount to an absence of absorption.

One attempt to solve these problems is described in U.S. Patent No. 4,361,546 wherein a core of dipyridamole and at least one mole of an acid substance per mole of dipyridamole is coated with a semi-permeable diffusion coating of a water-insoluble film former and a water-soluble polymer and optionally an acid-insoluble polymer.

Dipyridamole compositions containing at least 5 equivalents of an acid or acid substance per mole of dipyridamole to obtain a high degree of relative bioavailability have also been described.

U.S. Patent No. 4,367,217 describes spheroid particles of dipyridamole and at least one acid equivalent of an acid or acid substance per mole of dipyridamole coated with a dialysis membrane of acid-insoluble lacquers soluble in intestinal juices and uncoated particles in a capsule.

Object of the invention

It is an object of the invention to provide novel pH independent, controlled release oral tablets comprising an effective amount of an intimate mixture of dipyridamole or an acid addition salt thereof, at least 18 acid equivalents of at least one non-toxic, pharmaceutically acid or acid substance per mole of dipyridamole and a sufficient amount of a disintegration agent as specified below to allow at least 90% dissolution of dipyridamole in the gastrointestinal pH range of 1 to 7.

The invention

The novel compositions of the invention are pH independent, controlled release oral tablets comprising an effective amount of an intimate mixture of dipyridamole or an acid addition salt thereof, at least 18 acid equivalents of at least one non-toxic, pharmaceutically acid or acid substance per mole of dipyridamole and as a disintegration agent 5 to 10% by weight of sodium starch glycolate, 10 to 12% by weight of crospovidone or 3 to 5% by weight of croscarmelose, whereby at least 90% dissolution of dipyridamole in the gastrointestinal pH range of 1 to 7 is achieved.

Examples of non-toxic, pharmaceutically acceptable acids for the acid addition salts of dipyridamole are inorganic acids such as hydrochloric acid, hydrobromic acid, sulfuric acid, nitric acid and phosphoric acid and organic acids such as acetic acid, propionic acid, oxalic acid and benzene-sulfonic acid.

Examples of suitable solid acids for the compositions of the invention are non-toxic, pharmaceutically acceptable acids such as fumaric acid, maleic acid, tartaric acid, citric acid, succinic acid, ascorbic acid and mixtures thereof and acid salts such as sodium bisulfate, potassium bisulfate, betaine hydrochloride, monosodium and monopotassium salts of tartaric acid or citric acid and mixtures thereof.

The disintegration agents are sodium starch glycolate e.g. as sold under the name Primojel®, crospovidone e.g. as sold under the name Polyplasdone -XL® and croscarmelose e.g. as sold under the name AC-DI-SOL®. The amount of disintegrating agent will vary depending upon the specific agent selected but has to be sufficient to maintain continuously a highly localized acidic pH around the tablet to ensure sufficient release of dipyridamole regardless of the pH of the gastrointestinal environment but low enough so the tablet does not rapidly disintegrate into small particles which become rapidly neutralized rendering the dipyridamole insoluble.

The amount of sodium starch glycolate meeting these requirements has been found to be about 5 to 10%, preferably about 7.5% by weight of the tablet. With lower amounts such as 2% by weight, the tablet does not release an adequate amount of dipyridamole because the tablet does not disintegrate sufficiently to expose dipyridamole to the gastrointestinal tracts. With higher amounts on the order of 17.2% by weight, the tablet rapidly disintegrates into small particles and the localized acid pH around dipyridamole is lost whereby dipyridamole becomes insoluble in the gastrointestinal tracts.

Tests have shown that crospovidone should be used in a weight ratio of 10 to 12% and that croscarmelose should be used in an amount of 3 to 5% by weight of the tablet. The critical feature is that the tablet should disintegrate at a controlled rate so that at least 90% of the dipyridamole in the tablet will be dissolved and available for absorption by the gastrointestinal tracts of the warm-blooded animals both in the stomach and the intestine. In other words, the differing pH values of the stomach and intestinal tract will not effect the ability of dipyridamole to continuously dissolve.

The quantity of acid present in relation to the dipyridamole has no upper limit per se; it is only limited by the fact that if the quantity of acid present is too great, it is not possible to produce an oral form of

2

**0 121 901**

dipyridamole which can be easily swallowed. A ratio of at least 18 acid equivalents of acid present to 1 mol of dipyridamole is preferred.

Fumaric acid has proven to be a particularly suitable acid. It is physiologically non-toxic, and easy to compress, and when combined with dipyridamole, it does not produce a hydroscopic mixture. Its low solubility is essential to the invention; this ensures that, in the gastrointestinal tract the dipyridamole-containing particle is always surrounded by a sufficiently acidic microsphere in which the dipyridamole, which does not dissolve rapidly, is dissolved safely and completely.

In addition to the conventional excipients used, namely, polyvinylpyrrolidone, hydrogenated castor oil, and polyacrylic acid, it is also possible to use excipients such as methylcellulose, ethylcellulose, hydroxyethylcellulose, or hydroxypropyl methylcellulose. Furthermore, to achieve the desired release, mixtures consisting of dipyridamole and acidic substances may be granulated with fats dissolved in organic solvents or with lacquers resistant to gastric juices and then compressed into tablets.

In the following Examples there are described several preferred embodiments to illustrate the invention. However, it is to be understood that the invention is not intended to be limited to the specific embodiments.

Example 1

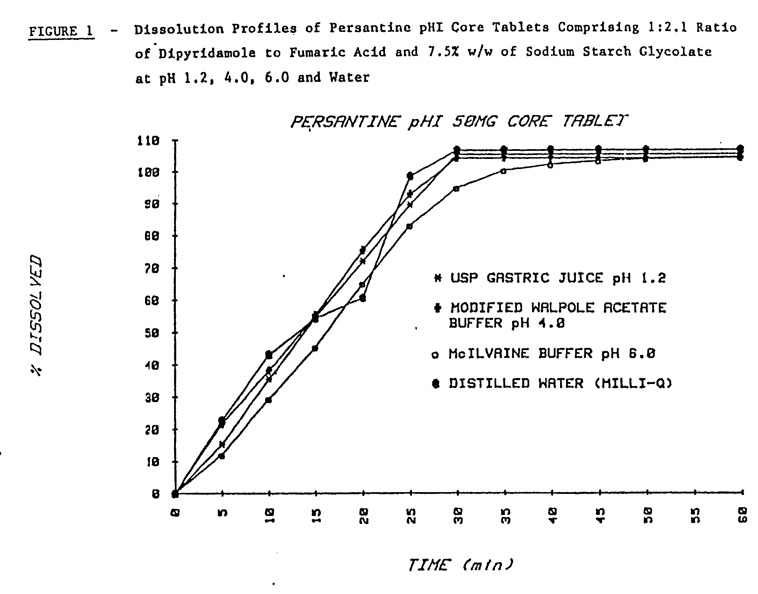

A mixture of 0.3 kg of dipyridamole, 0.63 kg of fumaric acid (mass ratio of 1:2.1), 0.0702 kg of polyvinyl-pyrrolidone, 0.3738 kg of lactose and 0.108 kg of sodium starch glycolate (7.2% by weight of the total composition) was wet granulated in admixture with isopropanol. The wet granulated was screened through an 18 mesh sieve and was dried at about 40°C. The dried granulate, 0.012 kg of magnesium stearate and 0.006 kg of colloidal fumed silicon dioxide were blended in a mixer and the mixture was compressed into 50 mg tablets in a tabletting machine. Using the USP XX paddle test method operating at 50 rpm at 37°C, the tablets showed a 100% dissolution in less than one hour at pH levels of 1.2 (0.1N HCl) 4.0 (Modified Walpole Acetate Buffer), 6.0 (McIlvaine Buffer) and water as can be seen from Fig. 1. The 100% dissolution is due to the fact that the proper mass ratio of dipyridamole to acid (1:2.1) and the proper amount of sodium starch glycolate (7.2% w/w) provide and maintain high microenvironmental acidic pH around the tablet until dipyridamole is released sufficiently.

Example 2

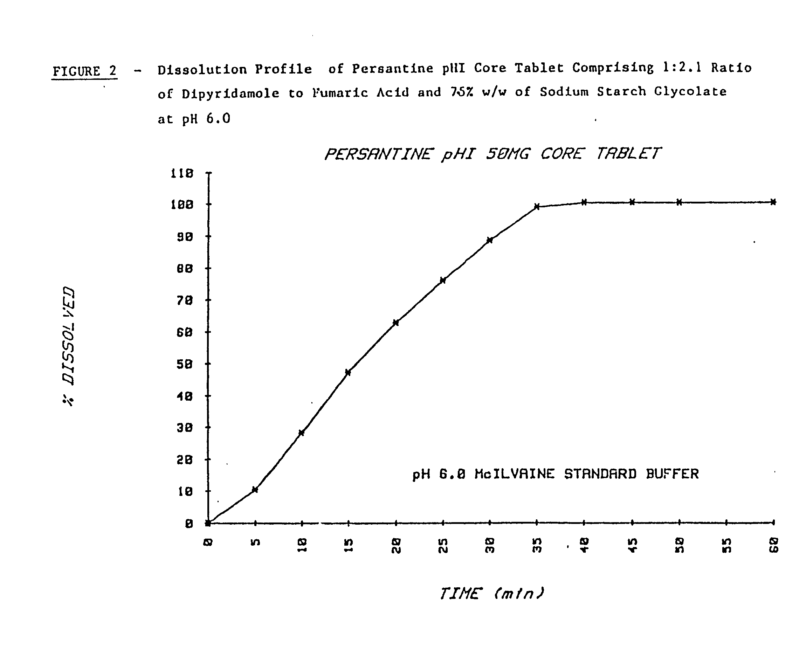

A mixture of 1.20 kg of dipyridamole, 2.52 kg of fumaric acid (1:2.1 by mass), 0.216 kg of polyvinylpyrrolidone, 0.466 kg of lactose and 0.360 kg of sodium starch glycolate (7.5% by weight of the total composition) was wet granulated with 95% ethanol and the wet granulate was milled in an oscillator and dried at 40°C. The dried granulate and 0.0384 kg of magnesium stearate were milled and blended and the mixture was compressed into 50 mg tablets. As can be seen from Fig. 2, the tablets showed 100% dissolution in one hour at a pH of 6 due to the fact that the proper mass ratio of dipyridamole to fumaric acid (1:2.1) and the proper amount of sodium starch glycolate (7.5% w/w) provide controlled disintegration and sufficient dissolution of dipyridamole.

Example 3

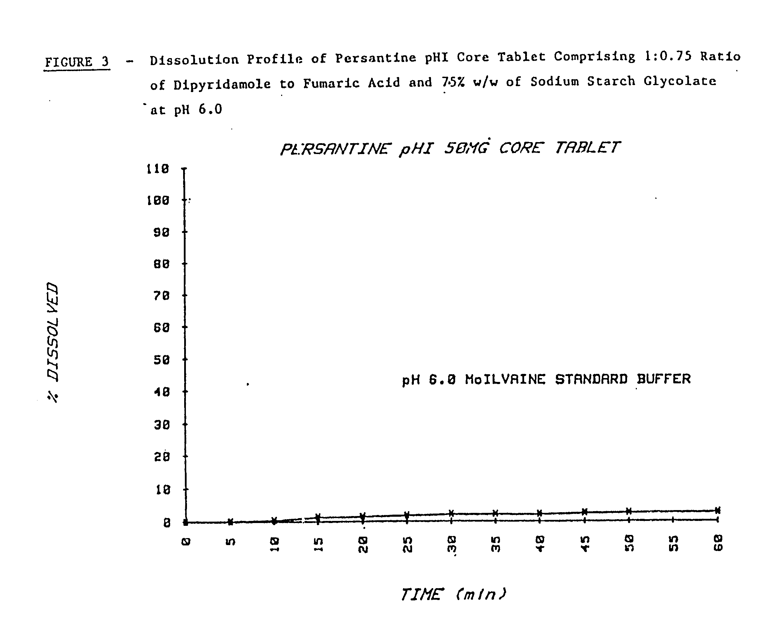

Using the procedure of Example 2, a mixture of 2.4 kg of dipyridamole, 1.790 kg of fumaric acid (1:0.75 by mass), 0.226 kg of polyvinylpyrrolidone, 0.346 kg of sodium starch glycolate (7.2% by weight of total composition) and 0.038 kg of magnesium stearate was formed into 50 mg tablets which showed only a 3% dissolution in one hour at a pH of 6.0 as seen from Fig. 3 due to the fact that there is an insufficient quantity of acid available thus not providing a low enough microenvironmental acidic pH.

Example 4

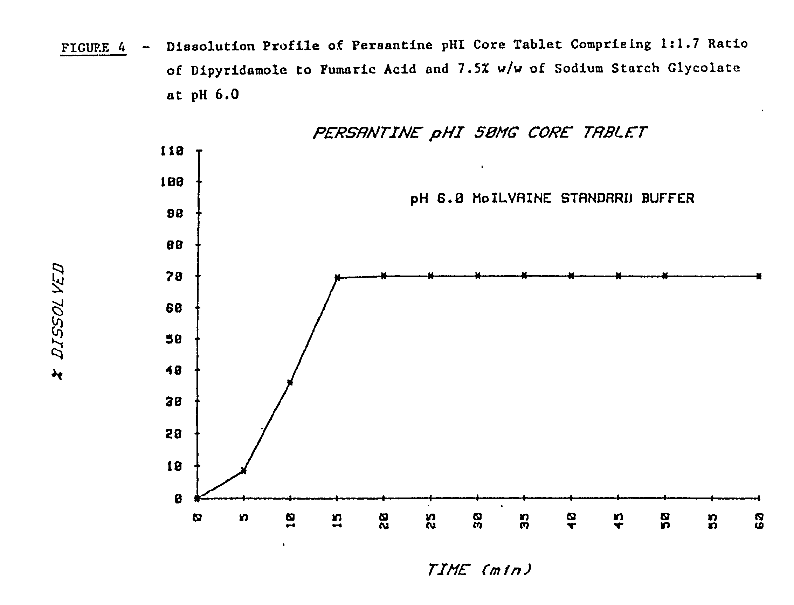

Using the procedure of Example 2, a mixture of 1.2 kg of dipyridamole, 2.04 kg of fumaric acid (1:1.7 by mass), 0.168 kg of polyvinylpyrrolidone, 0.994 kg of lactose, 0.360 kg of sodium starch glycolate (7.5% by weight of the total composition) and 0.038 kg of magnesium stearate was formulated into 50 mg tablets which as can be seen from Fig. 4 showed approximately 70% dissolution in one hour at a pH of 6.0 due to insufficient acid available thus providing too high a localized pH.

Example 5

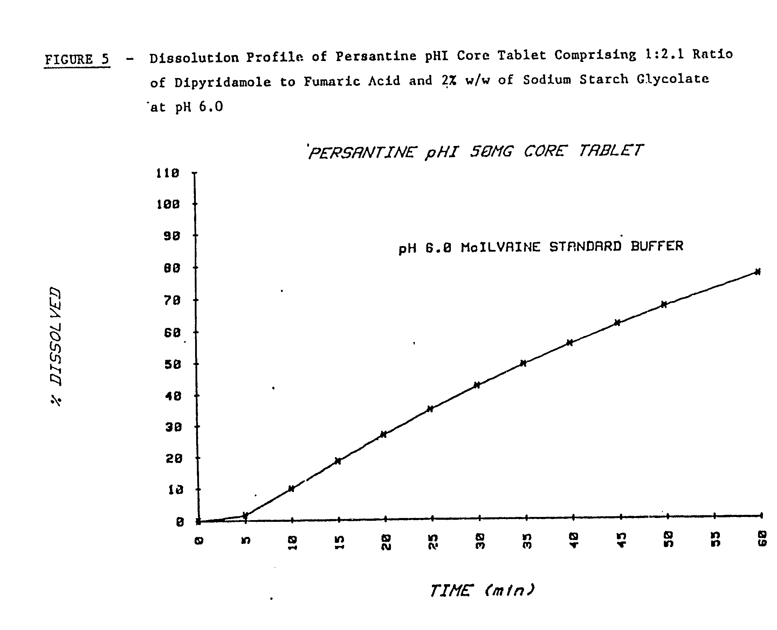

Using the procedure of Example 2, a mixture of 0.6 kg of dipyridamole, 1.26 kg of furmaric acid (1:2.1 by mass) 0.1404 kg of polyvinylpyrrolidone, 0.9036 kg of lactose, 0.06 kg of sodium starch glycolate (2% by weight of total composition) and 0.024 kg of magnesium stearate was formed into 50 mg tablets which can be seen from Fig. 5 showed approximately 80% dissolution in one hour at a pH of 6.0 due to prolonged disintegration time.

Example 6

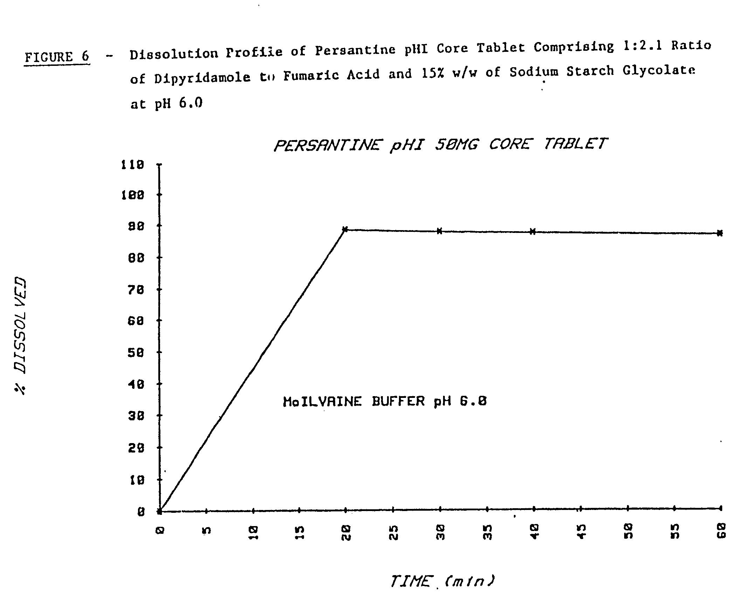

Using the procedure of Example 2, a mixture of 1.0 kg of dipyridamole, 2.1 kg of fumaric acid (1:2.1 by mass), 0.18 kg of polyvinylpyrrolidone, 0.688 kg (17.2% by weight) of sodium starch glycolate and 0.032 kg of magnesium stearate was formulated into 50 mg tablets which as can be seen from Fig. 6 showed approximately 86% dissolution in one hour at a pH of 6 due to the fact that the tablet disintegrated too rapidly.

Example 7

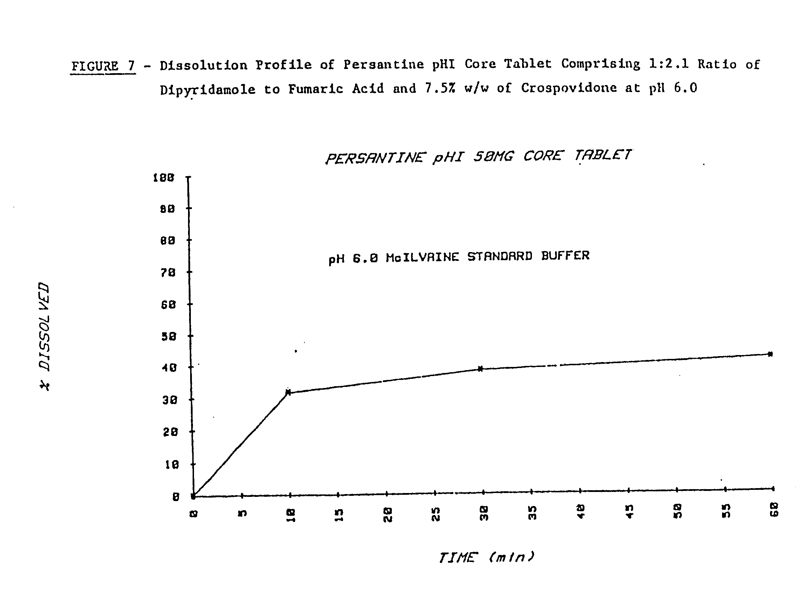

Using the procedure of Example 2, a mixture of 0.167 kg of dipyridamole 0.350 kg of fumaric acid (1:2.1 by mass), 0.030 kg of polyvinylpyrrolidone, 0.065 kg of lactose, 0.050 kg of crospovidone (7.5% by weight of the total composition) and 0.005 kg of magnesium stearate was formulated into 50 mg which as can be seen from Fig. 7 showed a 40% dissolution in one hour at a pH of 6.0 due to the fact that the tablet disintegrated too rapidly.

Example 8

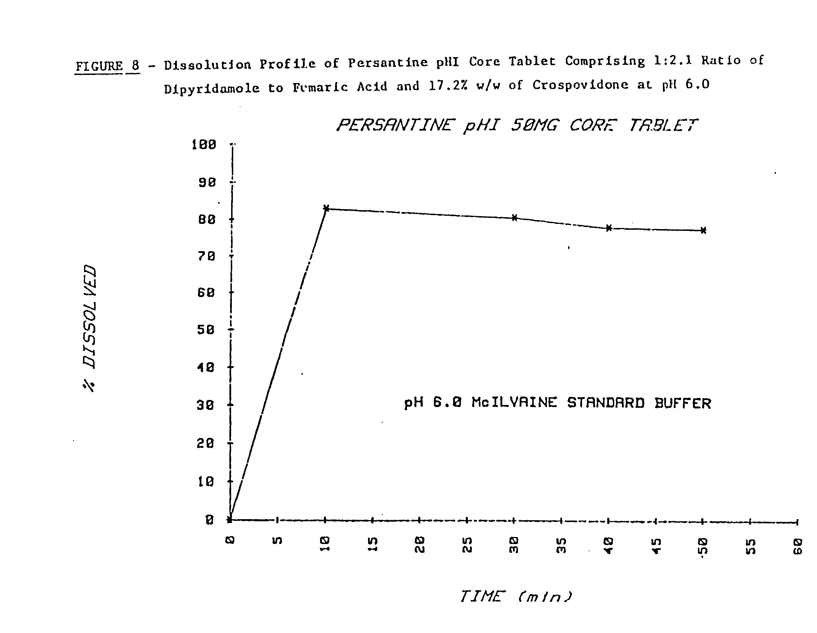

Using the procedure of Example 2, a mixture of 0.625 kg of dipyridamole, 1.313 kg of fumaric acid (1:2.1 by mass), 0.113 kg of polyvinylpyrrolidone, 0.430 kg of crospovidone (17.2% by weight of the total composition) and 0.02 kg of magnesium stearate was formulated into 50 mg tablets which as Fig. 8 illustrates showed a 80% dissolution in one hour at a pH of 6.0 due to the fact that the tablet disintegrated too rapidly.

Example 9

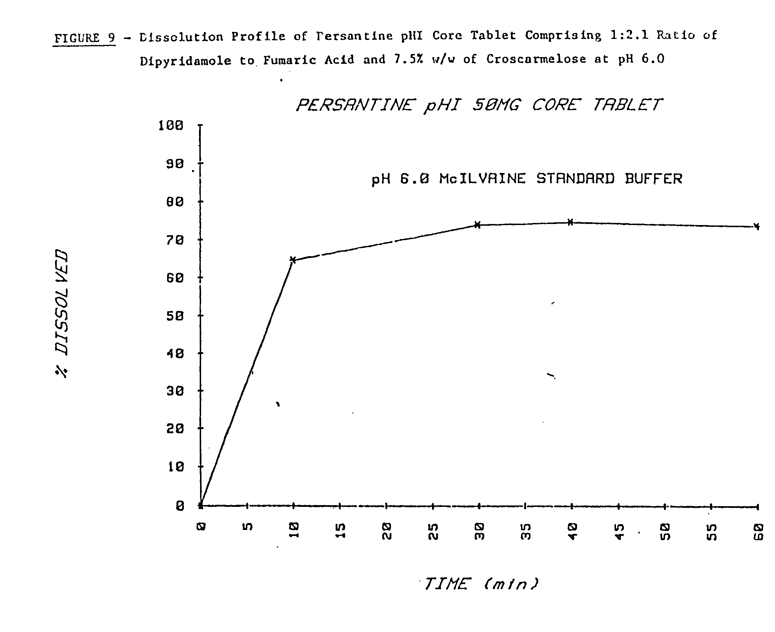

Using the procedure of Example 2, a mixture of 0.75 kg of dipyridamole, 1.575 kg of fumaric acid (1:2.1 by mass), 0.135 kg of polyvinylpyrrolidone, 0.291 kg of lactose, 0.225 kg of croscarmelose (7.5% by weight of the total composition) and 0.024 kg of magnesium stearate was formulated into 50 mg tablets which as seen from Fig. 9 showed a 75% dissolution in one hour at a pH of 6.0 due to the fact that the tablet disintegrated too rapidly.

**Claims for the Contracting States: BE, CH, DE, FR, GB, IT, LU, NL, SE**

1. A pH independent, controlled release oral tablet comprising an effective amount of an intimate mixture of dipyridamole or an acid addition salt thereof, at least 18 acid equivalents of at least one non-toxic, pharmaceutically acid or acid substance per mole of dipyridamole and as a disintegration agent 5 to 10% by weight of sodium starch glycolate, 10 to 12% by weight of crospovidone, or 3 to 5% by weight of croscarmelose, whereby at least 90% dissolution of dipyridamole in the gastrointestinal pH range of 1 to 7 is achieved.

2. A tablet of claim 1 wherein the acid is fumaric acid.

3. A tablet of claim 1 wherein the disintegration agent is sodium starch glycolate.

4. A tablet of claim 3 containing about 7.5% by weight of sodium starch glycolate.

**Claims (for the Contracting State: AT)**

1. Process for preparing a pH independent, controlled release oral tablet comprising dipyridamole, which comprises intimately mixing dipyridamole or an acid addition salt thereof, at least 18 acid equivalents of at least one non-toxic, pharmaceutically acid or acid substance per mole of dipyridamole and, as a disintegration agent 5 to 10% by weight of sodium starch glycolate, 10 to 12% by weight of crospovidone or 3 to 5% by weight of croscarmelose, and then forming said mixture into tablets, whereby said tablets have the property that at least 90% dissolution of dipyridamole in the gastrointestinal pH range of 1 to 7 is achieved.

2. Process as defined in claim 1, wherein said acid is fumaric acid.

3. Process as defined in claim 1, wherein the disintegration agent is sodium starch glycolate.

4. Process as defined in claim 3, wherein about 7.5% by weight of sodium starch glycolate is used.

**Patentansprüche für die Vertragsstaaten: BE, CH, DE, FR, GB, IT, LU, NL, SE**

1. Eine oral zu applizierende Tablette mit pH-unabhäniger, kontrollierter Freigabe, enthaltend eine wirksame Menge einer innigen Mischung aus Dipyridamol oder einem Säureadditionssalz hiervon und aus mindestens 18 Val mindestens einer nichttoxischen pharmazeutischen Säure oder sauren Substanz pro 1 Mol Dipyridamol und aus 5 bis 10 Gew.% Natrium-Stärkeglykolat, 10 bis 12 Gew.% Crospovidone oder 3 bis 5 Gew.% Croscaramelose als Zerfallsbeschleuniger, wodurch eine mindestens 90 %ige Freigabe des Dipyridamols im gastrointestinalen pH-Bereich zwischen 1 und 7 erreicht wird.

2. Eine Tablette gemäß Anspruch 1, worin die Säure als Fumarsäure vorliegt.

3. Eine Tablette gemäß Anspruch 1, worin der Zerfallsbeschleuniger als Natrium-Stärkeglykolat vorliegt.

4. Eine Tablette gemäß Anspruch 3, enthaltend ungefähr 7,5 Gew.% Natrium-Stärkeglykolat.

**Patentansprüche für den Vertragsstaat: AT**

1. Verfahren zur Herstellung einer oral zu applizierenden Tablette mit pH-unabhängiger kontrollierter Freigabe, enthaltend Dipyridamol, dadurch gekennzeichnet, daß Dipyridamol oder eines seiner Säureadditionssalze, mindestens 18 Val mindestens einer nichttoxischen, pharmazeutisch verwertbaren Säure oder sauren Substanz pro 1 Mol Dipyridamol und, als Zerfallsbeschleuniger, 5 bis 10 Gew.% Natrium-Stärkeglykolat, 10 bis 12 Gew.% Crospovidone oder 3 bis 5 Gew.% Croscaramelose innig miteinander vermischt werden und eine solche Mischung zu Tabletten geformt wird, wobei die so erhaltenen Tabletten die Eigenschaft haben, mindestens 90% des Dipyridamols in dem gastrointestinalen pH-Bereich von 1 bis 7 freizugeben.

2. Verfahren gemäß Anspruch 1, dadurch gekennzeichnet, daß besagte Säure Fumarsäure ist.

3. Verfahren gemäß Anspruch 1, dadurch gekennzeichnet, daß der besagte Zerfallsbeschleuniger Natrium-Stärkeglykolat ist.

4. Verfahren gemäß Anspruch 3, dadurch gekennzeichnet, daß 7,5 Gew.% Natrium-Stärkeglykolat verwendet werden.

**Revendications pour les Etats Contractants: BE, CH, DE, FR, GB, IT, LU, NL, SE**

1. Pastille orale à libération contrôlée indépendante du pH, comprenant une quantité efficace d'un mélange intime de dipyridamole ou de l'un de ses sels d'addition d'acides, au moins 18 équivalents acides d'au moins un acide ou une substance acide atoxique pharmaceutiquement par mole de dipyridamole et en tant qu'agent désagrégeant 5 à 10% en poids de glycolate d'amidon sodique, 10 à 12% en poids de crospovidone, ou 3 à 5% en poids de croscarmelose permettant d'obtenir une dissolution d'au moins 90% du dipyridamole dans le domaine du pH gastro-intestinal de 1 à 7.

2. Comprimé selon la revendication 1, dans lequel l'acide est l'acide fumarique.

3. Comprimé selon la revendication 1, dans lequel l'agent désagrégeant est le glycolate d'amidon sodique.

4. Comprimé selon la revendication 3 contenant environ 7,5% en poids de glycolate d'amidon sodique.

**Revendications pour l'Etat Contractant: AT**

1. Procédé pour la préparation d'une pastille orale à libération contrôlée indépendante du pH, comprenant du dipyridamole qui comprend les étapes consistant à mélanger intimement du dipyridamole ou l'un de ses sels d'addition d'acides, au moins 18 équivalents acides d'au moins un acide ou une substance acide atoxique pharmaceutiquement par mole de dipyridamole et en tant qu'agent désagrégeant 5 à 10% en poids de glycolate d'amidon sodique, 10 à 12% en poids de crospovidone, ou 3 à 5% en poids de croscarmelose et ensuite à formuler ledit mélange en pastilles, de façon que lesdites pastilles aient la propriété de permettre la dissolution d'au moins 90% du dipyridamole dans le domaine du pH gastro-intestinal de 1 à 7.

2. Procédé selon la revendication 1, dans lequel l'acide est l'acide fumarique.

3. Procédé selon la revendication 1, dans lequel l'agent désagrégeant est le glycolate d'amidon sodique.

4. Procédé selon la revendication 3, dans lequel on utilise environ 7,5% en poids de glycolate d'amidon sodique.

FIGURE 1  —  Dissolution Profiles of Persantine pHI Core Tablets Comprising 1:2.1 Ratio
of Dipyridamole to Fumaric Acid and 7.5% w/w of Sodium Starch Glycolate
at pH 1.2, 4.0, 6.0 and Water

PERSANTINE pHI 50MG CORE TABLET

% DISSOLVED

TIME (min)

* USP GASTRIC JUICE pH 1.2

✦ MODIFIED WALPOLE ACETATE
  BUFFER pH 4.0

o McILVAINE BUFFER pH 6.0

● DISTILLED WATER (MILLI-Q)

FIGURE 2 — Dissolution Profile of Persantine pHI Core Tablet Comprising 1:2.1 Ratio of Dipyridamole to Fumaric Acid and 7.5% w/w of Sodium Starch Glycolate at pH 6.0

## PERSANTINE pHI 50MG CORE TABLET

pH 6.0 McILVAINE STANDARD BUFFER

% DISSOLVED

TIME (min)

0 121 901

FIGURE 3 — Dissolution Profile of Persantine pHI Core Tablet Comprising 1:0.75 Ratio of Dipyridamole to Fumaric Acid and 7.5% w/w of Sodium Starch Glycolate at pH 6.0

PERSANTINE pHI 50MG CORE TABLET

pH 6.0 McILVAINE STANDARD BUFFER

% DISSOLVED

TIME (min)

FIGURE 4 — Dissolution Profile of Persantine pHI Core Tablet Comprising 1:1.7 Ratio of Dipyridamole to Fumaric Acid and 7.5% w/w of Sodium Starch Glycolate at pH 6.0

PERSANTINE pHI 50MG CORE TABLET

pH 6.0 McILVAINE STANDARD BUFFER

% DISSOLVED

TIME (min)

FIGURE 5 — Dissolution Profile of Persantine pHI Core Tablet Comprising 1:2.1 Ratio of Dipyridamole to Fumaric Acid and 2% w/w of Sodium Starch Glycolate at pH 6.0

PERSANTINE pHI 50MG CORE TABLET

pH 6.0 McILVAINE STANDARD BUFFER

% DISSOLVED

TIME (min)

PERSANTINE pHI 50MG CORE TABLET

% DISSOLVED

MoILVAINE BUFFER pH 6.0

TIME (min)

FIGURE 7 - Dissolution Profile of Persantine pHI Core Tablet Comprising 1:2.1 Ratio of Dipyridamole to Fumaric Acid and 7.5% w/w of Crospovidone at pH 6.0

PERSANTINE pHI 50MG CORE TABLET

pH 6.0 McILVAINE STANDARD BUFFER

% DISSOLVED

TIME (min)

FIGURE 8 – Dissolution Profile of Persantine pHI Core Tablet Comprising 1:2.1 Ratio of
Dipyridamole to Fumaric Acid and 17.2% w/w of Crospovidone at pH 6.0

PERSANTINE pHI 50MG CORE TABLET

pH 6.0 McILVAINE STANDARD BUFFER

% DISSOLVED

TIME (min)

0 121 901

8

FIGURE 9 — Dissolution Profile of Persantine pHI Core Tablet Comprising 1:2.1 Ratio of Dipyridamole to Fumaric Acid and 7.5% w/w of Croscarmelose at pH 6.0

PERSANTINE pHI 50MG CORE TABLET

pH 6.0 McILVAINE STANDARD BUFFER